# EUROPEAN PATENT SPECIFICATION

(11) **EP 0 756 870 B1**
(45) Date of publication and mention of the grant of the patent: **27.11.2002**
(21) Application number: 96111575.5
(22) Date of filing: 18.07.1996
(51) Int. Cl.: A61K 31/19, A61K 9/08, A61K 9/107, A61K 9/12

(54) **Pharmaceutical compositions for topic use on the basis of ketoprofen**
Pharmazeutische Zusammensetzungen zum topischen Gebrauch enthaltend Ketoprofen
Compositions pharmaceutiques à usage topique contenant du kétoprofène

(30) Priority: 03.08.1995 IT MI951720
(43) Date of publication of application: 05.02.1997
(73) Proprietor: Bayer S.p.A., 20156 Milano (IT)
(72) Inventor: Dondi, Gilberto, Cusano Milanino (MI) (IT)
(74) Representative: Klausner, Erich

(56) References cited:
- US-A- 4 704 406
- PATENT ABSTRACTS OF JAPAN vol. 016, no. 093 (C-0917), 6 March 1992 -& JP 03 275619 A (NITSUSUI SEIYAKU KK), 6 December 1991 -& DATABASE WPI Section Ch, Week 9204 Derwent Publications Ltd., London, GB; Class A12, AN 92-028853 XP002093383 & JP 03 275619 A (NISSUI SEIYAKU KK)

## Description

The topic treatment of rheumatic diseases or traumatic affections is often carried out using topic pharmaceutical formulations such as plasters, ointments, creams, gels, sprays and the like.

The aim of the topic treatment is to establish an analgesic and/or therapeutic effect.

The therapeutic effect is established depending on the penetration ability of the drug in the area suffering from the existing pathology.

It is generally accepted that the penetration route of a substance inside and through the skin is the skin itself and not other structures such as hair follicles or sudoriferous glands, although these may represent concomitant penetration routes.

There are well-founded reasons to believe that such structures do not constitute the prevalent routes; in humans, for example, palmar or plantar regions are provided with sudoriferous glands in an amount which is three times higher than in the other body regions; however, these regions are less capable of absorbing penetrating substances. Now, therefore, the prevailing opinion among the skilled in the art is that the transport of molecules of pharmacologically active substances occurs through passive diffusion and is strongly affected positively by skin hydration. Ointments which leave an occlusive film on the cutaneous surface produce hydration because of sweat accumulation at the skin-ointment interface and consequently they promote penetration.

Among the other factors affecting the absorbment is heat because it promotes a state of hyperthermia. Cold acts in the opposite way because it slows down the capillary circulation.

Among the many preparations available there are two types of spray formulations:
- compositions with a cooling effect which basically favors the analgesic action of the composition
- compositions with a revulsive/rubefacient effect which facilitates the blood supply with a consequent sensation of wellbeing.

US-A-4704406 refers to a sprayable preparation of ketoprofen, suitable for topic application, containing as solvent a mixture of one or more volatile solvents such as ethanol, propanol or isopropanol and one or more non volatile solvents such as propylene glycol, glycerin, a liquid polyethylene glycol or a polioxyalkylene glycol, in the weight ratio range of 1:1 to 20:1.

JP-A-3275619 refers to a topical composition containing a medicament, inter alia ketoprofen, a water-insoluble macromolecule, preferably a cellulose derivative, a plasticiser such as glycerol, glycerol ester, propylene glycol, polyethylene glycol, dimethyl (or diethyl) terephtalate, an organic solvent such as ethanol or propanol.

Recently, attempts have been made to use pharmacologically active substances generally employed parenterally and/or enterally. As a consequence, however, the need arose for agents capable of promoting the drug penetration through the skin.

In this connection, a number of different substances have been suggested such as alcohols, polyglycols, surfactants of different structures, without a common or clearly demonstrable result.

The more efficient system in order to assure the effectiveness of the pharmaceutical composition must be based on two types of action:
- a greater blood supply with an increase in skin permeability,
- a better penetration due to the presence of suitable selected agents.
The present invention refers to a pharmaceutical spray composition, for topic use, on the basis of ketoprofen, characterized in that it has the following percent composition, by weight:

| | |
|---|---|
| ketoprofen | 2 to 10 |
| alcohol | 40 to 80 |
| propylene glycol | 5 to 10 |
| poloxamer or polyoxyethylated castor oil | 1 to 10 |
| polyvinylpyrrolidone | 2 to 10 |
| ethanolamine | 0.9 to 4.4 |
| water | q.s. to 100 |

In particular, a sprayable pharmaceutical formulation, comprising an antirheumatic/analgesic active substance, such as ketoprofen, was developed, which formulation has the following percent composition (weight/weight):

| | |
|---|---|
| ketoprofen | 2-10 |
| isopropyl alcohol | 40-80 |
| benzyl nicotinate | 0.2-2 |
| propylene glycol | 5-10 |
| poloxamer | 1-10 |
| polyvinylpyrrolidone | 2-10 |
| ethanolamine | 0.9-4.4 |
| water | q.s. to 100 |

In the above-mentioned formulation, benzyl nicotinate is the rubefacient agent which, as it is known to the skilled in the art, can be substituted with other substances such as, e.g., camphor, menthol, capsicum, or mixtures thereof; propylene glycol and isopropyl alcohol, or other alcohols commonly used in art of drugs for topic use, are the principal carriers, poloxamer (ethylene-polyoxypropylene block copolymer), which may be substituted by an equal amount of polyoxyethylated castor oil, acting as a penetration agent, and polyvinylpyrrolidone is added as a film forming agent in order to confer adhesive properties to the skin and to favor the absorption of the topic composition of the present invention.

A critical parameter of the present formulation is the pH value of the ketoprofen solution in the hydroalcoholic carrier in order to give stability to the pharmaceutical composition. The best results were obtained for pH values between 5 and 7. According to the present invention these values can be obtained by adding suitable neutralizing agents such as, e.g. ethanolamine, lysine, triethanolamine, methylglucamine and, generally, any suitable amine of the kind used for the manufacture of pharmaceutical compositions.

The manufacture of ketoprofen spray solutions is carried out by dissolving the ingredients in a previously prepared alcohol-water mixture. Finally, ethanolamine is added and the obtained pH value is checked and adjusted, if necessary.

Then the composition is filled up in glass or pharmacologically inert plastic bottles, such as, e.g., opaque, white polyethylene, equipped with a mechanical pump for spraying pharmaceutical compositions for topic use in an exactly dosed amount.

The present invention is better illustrated by the following examples. A person skilled in the art of pharmaceutical compositions will, without any difficulty, be able to adapt the process parameteres to the particular needs without, thereby, departing from the gist of the invention.

### Example 1

To a solution of:

| | |
|---|---|
| isopropyl alcohol | 69.5 g |
| purified water | 10.0 g |

the following ingredients are added under stirring:

| | |
|---|---|
| poloxamer 407 | 2.0 g |
| menthol | 0.2 g |
| propylene glycol | 8.0 g |
| benzyl nicotinate | 0.2 g |
| polyvinylpyrrolidone 25 | 4.0 g |
| ketoprofen | 5.0 g |

To the so obtained homogeneous mixture

| | |
|---|---|
| ethanolamine | 1.1 g |

is added; the final pH value of the preparation is 6.5.

Then the solution is divided and packed in dark glass or white polyethylene bottles, equipped with a mechanical sprayer.

### Example 2

To a solution of:

| | |
|---|---|
| ethyl alcohol | 68.9 g |
| purified water | 7.0 g |

the following ingredients are added, under stirring:

| | |
|---|---|
| poloxamer 407 | 2.0 g |
| propylene glycol | 10.0 g |
| benzyl nicotinate | 2.0 g |
| polyvinylpyrrolidone 25 | 4.0 g |
| ketoprofen | 5.0 g |

To the so prepared mixture

| | |
|---|---|
| ethanolamine | 1.1 g |

is added; the final pH value of the preparation is 6.5.

### Example 3

To a solution of:

| | |
|---|---|
| ethyl alcohol | 68.9 g |
| purified water | 7.0 g |

the following ingredients are added, under stirring:

| | |
|---|---|
| polyoxyethylated castor oil | 2.0 g |
| propylene glycol | 10.0 g |
| benzyl nicotinate | 2.0 g |
| polyvinylpyrrolidone 25 | 4.0 g |
| ketoprofen | 5.0 g |

To the so prepared mixture

| | |
|---|---|
| ethanolamine | 1.1 g |

is added; the final pH value of the preparation is 6.5.

## Claims

1. A pharmaceutical spray composition, for topic use, on the basis of ketoprofen, **characterized in that** it has the following percent composition, by weight:
| | |
|---|---|
| ketoprofen | 2 to 10 |
| alcohol | 40 to 80 |
| propylene glycol | 5 to 10 |
| poloxamer or polyoxyethylated castor oil | 1 to 10 |
| polyvinylpyrrolidone | 2 to 10 |
| ethanolamine | 0.9 to 4.4 |
| water | q.s. to 100 |

2. A pharmaceutical spray composition for topic use, according to claim 1, **characterized in that** its pH value is in the range of from 5 and 7.

3. A pharmaceutical spray composition for topic use, according to claims 1 and 2, **characterized by** the following percent composition, in parts by weight:
| | |
|---|---|
| ketoprofen | 5.0 |
| isopropyl alcohol | 69.5 |
| purified water | 10.0 |
| poloxamer 407 | 2.0 |
| menthol | 0.2 |
| propylene glycol | 8.0 |
| benzyl nicotinate | 0.2 |
| polyvinylpyrrolidone 25 | 4.0 |
| ethanolamine | 1.1 |
and a pH value of 6.5

4. A pharmaceutical spray composition, for topic use, according to claims 1 and 2, **characterized by** the following percent composition, in parts by weight:
| | |
|---|---|
| ketoprofen | 5.0 |
| ethyl alcohol | 68.9 |
| purified water | 7.0 |
| poloxamer | 2.0 |
| propylene glycol | 10.0 |
| benzyl nicotinate | 2.0 |
| polyvinylpyrrolidone 25 | 4.0 |
| ethanolamine | 5.0 |
and a pH value of 6.5

5. A process for manufacturing a pharmaceutical spray composition, for topic use, on the basis of ketoprofen according to anyone of the preceding claims, **characterized in that** the ketoprofen is dissolved in a previously prepared alcohol-purified water solution, followed by adding the other components of the composition, under continuous stirring, adjusting the pH to a value in the range of 5 and 7, with a pharmaceutically acceptable amine neutralizing agent.

## Patentansprüche

1. Pharmazeutische Spray-Zusammensetzung für die topische Anwendung auf der Basis von Ketoprofen, **dadurch gekennzeichnet, dass** sie die folgende Zusammensetzung In Gew.-% aufweist:
| | |
|---|---|
| Ketoprofen | 2 bis 10 |
| Alkohol | 40 bis 80 |
| Propylenglykol | 5 bis 10 |
| Poloxamer oder polyoxyethyliertes Kastoröl | 1 bis 10 |
| Polyvinylpyrrolidon | 2 bis 10 |
| Ethanolamin | 0,9 bis 4,4 |
| Wasser | q,s, auf 100 |

2. Pharmazeutische Spray-Zusammensetzung für die topische Anwendung nach Anspruch 1, **dadurch gekennzeichnet, dass** ihr pH-Wert im Bereich von 5 bis 7 liegt.

3. Pharmazeutische Spray-Zusammensetzung für die topische Anwendung nach den Ansprüchen 1 und 2, **dadurch gekennzeichnet, dass** sie die folgende prozentuale Zusammensetzung in Gewichtsteilen aufweist;
| | |
|---|---|
| Ketoprofen | 5,0 |
| Isopropylalkohol | 69,5 |
| gereinigtes Wasser | 10,0 |
| Poloxamer 407 | 2,0 |
| Menthol | 0,2 |
| Propylenglykol | 8,0 |
| Benzylnikotinat | 0,2 |
| Polyvinylpyrrolidon 25 | 4,0 |
| Ethanolamin | 1,1 |
| und ein pH-Wert von | 6,5. |

4. Pharmazeutische Spray-Zusammensetzung zur topischen Anwendung nach Anspruch 1 und 2, **dadurch gekennzeichnet, dass** sie folgende prozentuale Zusammensetzung In Gewichtsteilen aufweist:
| | |
|---|---|
| Ketoprofen | 5,0 |
| Ethylalkohol | 68,9 |
| gereinigtes Wasser | 7,0 |
| Poloxamer | 2,0 |
| Propylenglykol | 10,0 |
| Benzylnikotinat | 2,0 |
| Polyvinylpyrrolidon 25 | 4,0 |
| Ethanolamin | 5,0 |
| und ein pH-Wert von | 6,5. |

5. Verfahren zur Herstellung einer pharmazeutischen Spray-Zusammensetzung zur topischen Anwendung auf der Basis von Ketoprofen nach irgend einem der vorhergehenden Ansprüche, **dadurch gekennzeichnet, dass** das Ketoprofen in einer zuvor hergestellten Alkohol/gereinigtes Wasser-Lösung gelöst wird, anschlleßend die anderen Komponenten der Zusammensetzung unter kontinuierlichem Rühren zugegeben werden, und der pH-Wert auf einen Wert im Bereich von 5 bis 7 mit einem pharmazeutisch verträglichen Amin-Neutralisierungsmittel eingestellt wird.

## Revendications

1. Composition pharmaceutique en spray à usage topique à base de kétoprofène, **caractérisée en ce qu'**elle présente la composition suivante exprimée en pourcentage en poids :
| | |
|---|---|
| Kétoprofène | de 2 à 10 |
| Alcool | de 40 à 80 |
| Propylèneglycol | de 5 à 10 |
| Poloxamère ou huile de ricin polyoxyéthylée | de 1 à 10 |
| Polyvinylpyrrolidone | de 2 à 10 |
| Ethanolamine | de 0,9 à 4,4 |
| Eau | autant que nécessaire jusqu'à la totalité |

2. Composition pharmaceutique en spray à usage topique selon la revendication 1, **caractérisée en ce que** sa valeur de pH est comprise dans la plage allant de 5 à 7.

3. Composition pharmaceutique en spray à usage topique selon les revendications 1 et 2, **caractérisée par** la composition suivante exprimée en parties en pourcentage en poids :
| | |
|---|---|
| Kétoprofène | 5,0 |
| Alcool isopropylique | 69,5 |
| Eau purifiée | 10,0 |
| Poloxamère 407 | 2,0 |
| Menthol | 0,2 |
| Propylèneglycol | 8,0 |
| Nicotinate de benzyle | 0,2 |
| Polyvinylpyrrolidone 25 | 4,0 |
| Ethanolamine | 1,1 |
et une valeur de pH de 6,5.

4. Composition pharmaceutique en spray à usage topique selon les revendications 1 et 2, **caractérisée par** la composition suivante exprimée en parties en pourcentage en poids :
| | |
|---|---|
| Kétoprofène | 5,0 |
| Alcool éthylique | 68,9 |
| Eau purifiée | 7,0 |
| Poloxamère | 2,0 |
| Propylèneglycol | 10,0 |
| Nicotinate de benzyle | 2,0 |
| Polyvinylpyrrolidone 25 | 4,0 |
| Ethanolamine | 5,0 |
et une valeur de pH de 6,5.

5. Procédé de préparation d'une composition pharmaceutique en spray, à usage topique, à base de kétoprofène selon l'une quelconque des revendications précédentes, **caractérisé en ce que** le kétoprofène est dissous dans une solution préalablement préparée à base d'alcool et d'eau purifiée, suivi par l'ajout des autres composants de la composition, sous agitation continue, en ajustant le pH à une valeur comprise dans la plage allant de 5 à 7, avec un agent de neutralisation à base d'amines pharmaceutiquement acceptable.
